# EUROPÄISCHE PATENTANMELDUNG

(11) **EP 1 174 105 A2**
(43) Veröffentlichungstag der Anmeldung: **23.01.2002**
(21) Anmeldenummer: 01117431.5
(22) Anmeldetag: 19.07.2001
(51) Int. Cl.: A61F 17/00

(54) **Nothilfepackung**

(30) Priorität: 22.07.2000 DE 20012747 U
(71) Anmelder: FRANZ KALFF GmbH, D-53881 Euskirchen (DE)
(72) Erfinder: Willkomm, Monika, 54595 Prüm (DE)
(74) Vertreter: Selting, Günther, Dipl.-Ing.

(57) **Zusammenfassung**

Eine Nothilfepackung (10) zur Aufnahme von Erste-Hilfe-Mitteln weist einen flexiblen Rücken (12) auf, der auf seiner Innenseite Taschen (26,28,30) für die Erste-Hilfe-Mittel aufweist und der in mindestens zwei Faltabschnitte (11₁-11₅) unterteilt ist. Zwei aneinander angrenzende Faltabschnitte sind buchartig zusammenklappbar und bilden zusammengeklappt eine Schutzmappe, in deren Innenraum die Taschen (26,28,30) geschützt angeordnet sind. Der Rücken (12) ist von einer durchgehenden Rückenbahn gebildet, wobei das Rückenbahnmaterial von dem Taschenbahnmaterial verschieden ist. Die Taschen (26,28,30) sind auf der einen Seite von der Rückenbahn (14) und auf der anderen Seite von mindestens einer Taschenbahn (16,18) begrenzt. Durch die Wahl unterschiedlicher Materialien für die Taschenbahn und die Rückenbahn wird eine hohe Festigkeit für die gesamte Nothilfepackung gewährleistet und eine robuste Schutzmappe zum Schutz der Erste-Hilfe-Mittel innerhalb der Schutzmappen-Faltabschnitte sichergestellt.

## Beschreibung

Die Erfindung bezieht sich auf eine Nothilfepackung zur Aufnahme von Erste-Hilfe-Mitteln.

Nothilfepackungen dienen der Aufbewahrung und Bereithaltung von Erste-Hilfe-Mitteln in Kraftfahrzeugen, im Haushalt oder in anderen Bereichen. Nothilfepackungen können beispielsweise aus dünnen Kunststofffolien bestehen, die miteinander verschweißt sind und Taschen zur Aufnahme der Erste-Hilfe-Mittel bilden. Derartige Nothilfepackungen sind in erster Linie Ordnungsmittel und werden zum Schutz gegen Schmutz, Feuchtigkeit, Licht und mechanischer Beschädigung in steifen Kunststoffkästen gelagert. Der steife Kunststoffkasten erfordert zur Unterbringung relativ viel Platz, ist aufwendig in der Herstellung und umständlich in der Handhabung. Aus DE 295 01 305 ist eine Nothilfepackung bekannt, die zum Schutz der in Kunststofffolie untergebrachten Tascheninhalte eine separate Tasche aus einem Textilgewebe aufweist. Die aus der Tasche ausgeklappten Folienabschnitte bieten keinen guten Schutz der Tascheninhalte bei einer Unterlage mit scharfkantigen Steinen etc. Aus DE 93 16 351 U1 ist eine Nothilfepackung bekannt, die einen flexiblen Rücken mit Taschen für Erste-Hilfe-Mittel auf der Innenseite aufweist. Der Rücken ist in vier Faltabschnitte unterteilt, wobei zwei aneinander angrenzende Faltabschnitte buchartig zu einer Schutzmappe zusammenklappbar sind. Die beiden anderen Faltabschnitte sind in die beiden Schutzmappen-Faltabschnitte einklappbar. Die Schutzmappen-Faltabschnitte weisen eine doppellagige Rückenbahn auf, während die in die Schutzmappe eingeklappten Faltabschnitte eine einlagige Rückenbahn aufweisen. Die äußeren Längsrandstreifen der Rückenbahn sind nach innen umgeschlagen und durch Quernähte in mehrere Taschen unterteilt. Die Rückenbahn und die Taschenbahn sind aus demselben Material, so dass die Rückenbahn insbesondere in ihrem einlagigen Abschnitt keinen starken mechanischen Schutz der Erste-Hilfe-Mittel in den Taschen bietet.

Aufgabe der Erfindung ist es, eine einfach aufgebaute und guten Schutz für die Tascheninhalte bietende Nothilfepackung zu schaffen.

Diese Aufgabe wird erfindungsgemäß mit den Merkmalen des Anspruchs 1 gelöst.

Die erfindungsgemäße Nothilfepackung weist einen Rücken auf, der von einer durchgehenden Rückenbahn gebildet wird. Die Taschen werden auf der einen Seite von der Rückenbahn und auf der anderen Seite von mindestens einer separaten Taschenbahn begrenzt, wobei das Taschenbahnmaterial verschieden von dem Rückenbahnmaterial ist. Die Nothilfepackung wird über alle Faltabschnitte nur durch eine Rückenbahn und eine separate Taschenbahn gebildet, die auf der Rückenbahn befestigt ist. Das Rückenbahnmaterial kann stärker und mechanisch fester als das Taschenbahnmaterial ausgebildet sein. Die Nothilfepackung kann auf diese Weise aus nur zwei Materiallagen hergestellt werden. Gleichzeitig ist sichergestellt, dass die von der Rückenbahn gebildete Schutzmappe aus einem widerstandsfähigen Material gebildet ist, das Schutz gegen Flüssigkeiten, Schmutz, Licht und mechanischer Beschädigung bietet. Dadurch ist eine kastenlose Lagerung der Erste-Hilfe-Mittel möglich. Auch wenn alle Faltabschnitte aufgeklappt sind, sind die Erste-Hilfe-Mittel in allen Taschen durch die robuste Rückenlage gut gegen eine feuchte oder schmutzige Unterlage geschützt, auch in den Taschen der Faltabschnitte, die aus der Schutzmappe herausgeklappt sind.

Da die Nothilfepackung aus nur zwei Materiallagen gebildet wird, ist sie sehr einfach und damit sehr preiswert herstellbar. Durch die Wahl unterschiedlicher Materialien für die Taschenbahn und die Rückenbahn wird eine hohe Festigkeit für die gesamte Nothilfepackung gewährleistet und insbesondere eine robuste Schutzmappe zum Schutz der Erste-Hilfe-Mittel innerhalb der Schutzmappen-Faltabschnitte sichergestellt.

Gemäß einer bevorzugten Ausgestaltung bilden die Rückenbahn und die Taschenbahn mindestens drei Faltabschnitte, wobei zwischen die beiden Schutzmappen-Faltabschnitte die übrigen Faltabschnitte einklappbar sind. Die Rückenbahn und die Taschenbahn erstrecken sich über alle Faltabschnitte jeweils einstückig und einlagig, also sowohl über die beiden Schutzmappen-Faltabschnitte als auch über die übrigen Faltabschnitte, die zwischen die Schutzmappen-Faltabschnitte eingeklappt werden. Dadurch ist auch die Herstellung von Nothilfepackungen, die aus drei, vier, fünf oder mehr Faltabschnitten bestehen, einfach und preiswert realisierbar.

Vorzugsweise wird der Rücken durch die durchgehend einlagige Rückenbahn gebildet, und die Taschenbahn ist in Längsrichtung durchgehend und einlagig ausgebildet und auf der Innenseite der Rückenbahn linienförmig befestigt. Die Taschen werden also nicht von mehreren nebeneinander liegenden Taschenbahnen gebildet, sondern von einer einzigen und in Längsrichtung durchgehenden Taschenbahn. Die Taschenbahn wird auf der Rückenbahn linienförmig befestigt, beispielsweise genäht, geschweißt, geklebt, geklammert oder genietet. Durch die linienförmige Befestigung der Taschenbahn auf der Rückenbahn werden die Taschen auf einfache Weise gebildet. Beim Vernähen der Taschenbahn mit der Rückenbahn wird eine zuverlässige Verbindung geschaffen, die einfach herstellbar ist.

Gemäß einer bevorzugten Ausgestaltung ist eine zweite Taschenbahn in Längsrichtung parallel und gegenüberliegend der ersten Taschenbahn vorgesehen, wobei die Taschenöffnungen der Taschen beider Taschenbahnen zur Längsmittelachse der Rückenbahn orientiert und einander gegenüberliegend angeordnet sind. Jede Taschenbahn bedeckt ungefähr zwei Fünftel der Rückenbahnbreite über die gesamte Länge der Rückenbahn. Die Taschenbahnen können aber auch jeweils annähernd die Hälfte der Breite der Rückenbahn bedecken, so dass sich ihre Öffnungen unmittelbar gegenüberliegen. Die Öffnungen der von den Taschenbahnen begrenzten Taschen weisen jeweils zur Mitte, so dass Erste-Hilfe-Mittel aus den Taschen nicht herausfallen können. Die jeweils einander gegenüberliegenden Taschen können miteinander fluchten, können jedoch auch versetzt zueinander und von verschiedener Länge sein. Die Tiefe der Taschen, d. h. die Breite der beiden Taschenbahnen ist gleich, kann jedoch auch verschieden voneinander sein. Dadurch ergeben sich große Gestaltungsspielräume bei der Anordnung, Verteilung und Größe der Taschen für die Erste-Hilfe-Mittel, wie dies für die Unterbringung der unterschiedlichsten Erste-Hilfe-Mittel in deren zahlreichen maßlichen Varianten erforderlich ist.

Vorzugsweise besteht die Rückenwand aus einem textilen Gewebe, insbesondere aus einem Kunstfasergewebe. Derartige Gewebe bieten einen guten Schutz gegen Wasser, Schmutz, Licht und mechanische Beschädigungen der Erste-Hilfe-Mittel in den Taschen, sind preiswert erhältlich und einfach zu verarbeiten.

Vorzugsweise besteht die Taschenbahn aus einer einlagigen Kunststofffolie. Die Kunststofffolie kann ferner transparent und/oder bedruckt sein. Die von einer Kunststofffolie gebildete Taschenbahn ist bei ausreichender Festigkeit preiswert und einfach zu verarbeiten.

Vorzugsweise sind im Bereich der Vernähungen der Taschenbahn mit der Rückenbahn Verstärkungsstreifen zur Verstärkung der Vernähungen vorgesehen. Die Verstärkungsstreifen bestehen aus einem Material, das eine hohe Festigkeit gegen Einreißen gewährleistet. Die Verstärkungsstreifen werden im Bereich der Vernähungen auf die Taschenbahn gelegt und anschließend mit der Taschenbahn und der Rückenbahn zusammengenäht.

Vorzugsweise ist ein Reißverschluss vorgesehen, der die zwei zur Schutzmappe zusammengefalteten Rückenbahn-Faltabschnitte an den drei offenen Seiten miteinander verbindet, so dass alle drei offenen Seiten der Schutzmappe mit dem Reißverschluss verschließbar sind. Der Reißverschluss wird vorzugsweise an der Rückseite, d. h. außen der Rückenbahn angebracht, wobei die beiden Reißverschlusshälften zusammen ungefähr einen geschlossenen Kreis bilden. Auf diese Weise ist der Reißverschluss einfach mit der Rückenbahn vernähbar und gewährleistet einen allseitigen Verschluss der beiden zusammengefalteten Faltabschnitte, die eine Schutzmappe bilden. Als Verschluss kann auch ein Klettverschluss oder Druckknopfverschluss dienen. Der Verschluss kann auch laschenartig ausgebildet sein, d. h. die beiden Faltabschnitte, die die Schutzmappe bilden, werden nur durch eine Lasche zusammengehalten, nicht aber rundum verschlossen.

Vorzugsweise ist die Gewebe-Rückenbahn auf ihrer Innenseite mit einer flüssigkeitsundurchlässigen Kunststoffbeschichtung versehen. Dadurch wird das Eindringen von Feuchtigkeit in die Taschen und damit in die Erste-Hilfe-Mittel in den Taschen vermieden.

Im Folgenden werden unter Bezugnahme auf die Zeichnungen vier Ausführungsbeispiele der Erfindung näher erläutert.

Es zeigen:
- Fig. 1: eine erste Ausführungsform einer Nothilfepackung in Draufsicht auf die Innenseite,
- Fig. 2: die Nothilfepackung der Fig. 1 im Längsschnitt,
- Fig. 3: die Nothilfepackung der Fig. 1 im Längsschnitt beim Zusammenklappen,
- Fig. 4: die Nothilfepackung der Fig. 1 in Seitenansicht im zusammengeklapptem Zustand,
- Fig. 5: die Nothilfepackung der Fig. 1 im vollständig geschlossenen Zustand,
- Fig. 6: eine Detaildarstellung der Nothilfepackung der Fig. 1,
- Fig. 7: eine zweite Ausführungsform einer Nothilfepackung mit, gegenüber der Nothilfepackung der Fig. 1, anderer Aufteilung der Taschen,
- Fig. 8: eine dritte Ausführungsform einer Nothilfepackung mit zwei Faltabschnitten,
- Fig. 9: ein Längsschnitt der Nothilfepackung der Fig. 8,
- Fig. 10: eine perspektivische Darstellung der geschlossenen Nothilfepackung der Fig. 8,
- Fig. 11: eine Draufsicht einer vierten Ausführungsform einer Nothilfepackung mit Seitenwände der Faltmappe bildenden Randstreifen, und
- Fig. 12: eine perspektivische Darstellung der geschlossenen Nothilfepackung der Fig. 11.

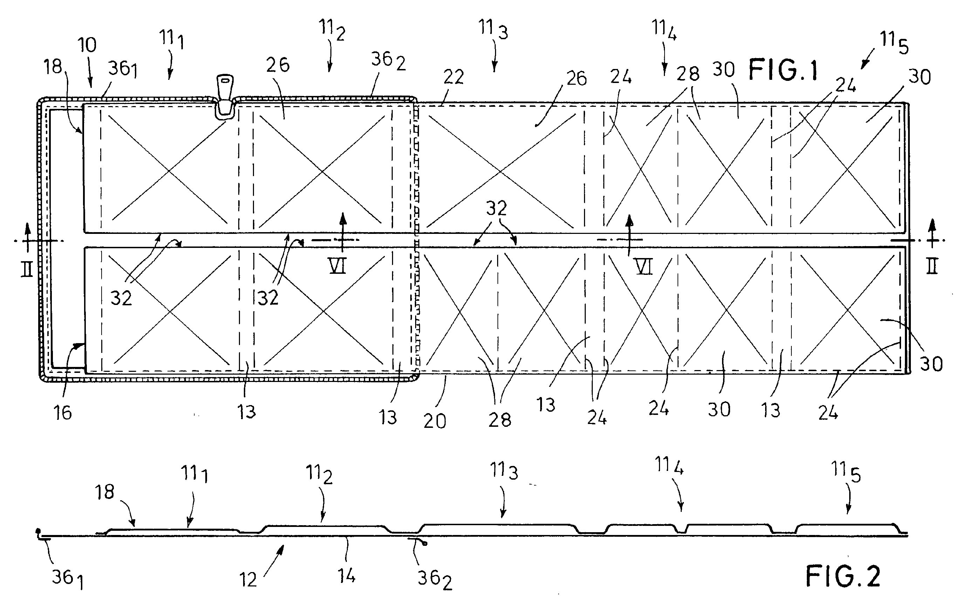

In den Fign. 1 - 12 sind vier verschiedene Nothilfepackungen 10, 10', 50, 80 dargestellt, die der geordneten Aufnahme und dem Schutz von Erste-Hilfe-Mitteln, insbesondere von Verbandszeug, dienen. Derartige Nothilfepackungen 10, 10', 50, 80 werden insbesondere in Kraftfahrzeugen und im Haushalt verwendet.

Die Nothilfepackung 10 der Fig. 1 - 6 weist einen Rücken 12 auf, der von einer einstückigen und durchgehend einlagigen Rückenbahn 14 gebildet wird. Die Rückenbahn 14 besteht aus einem Kunstfasergewebe, insbesondere aus einem Nylon-Gewebe. Die Rückenbahn 14 ist auf ihrer Innenseite, d. h. auf der Seite der Taschen 26, 28, 30 mit einer flüssigkeitsundurchlässigen Kunststoffbeschichtung versehen. Dadurch wird das Eindringen von Feuchtigkeit durch die Rückenbahn in die Taschen 26, 28, 30 vermieden.

Auf der Innenseite der Rückenbahn 14 sind zwei Taschenbahnen 16, 18 befestigt, die jeweils in Längsrichtung durchgehend und einlagig ausgebildet sind. Die Taschenbahnen 16, 18 bestehen aus einer transparenten Kunststofffolie aus PELD. Das Taschenbahnmaterial ist dünner als das Rückenbahnmaterial. Die Taschenbahnen 16, 18 sind auf die Rückenbahn 14 aufgenäht. Jede Taschenbahn 16, 18 ist zum einen in Längsrichtung am jeweiligen Längsrand der Rückenbahn 14 durch eine Längsnaht 20, 22 über die gesamte Länge und mit einer Vielzahl von Quernähten 24 jeweils über die gesamte Breite einer Taschenbahn 16, 18 mit der Rückenbahn 14 vernäht. Die Taschenbahn kann auch durch Verschweißungen oder durch andere linienartige Verbindungen mit der Rückenbahn verbunden sein. Die transparenten oder eingefärbten Taschenbahnen 16, 18 sind im Bereich der Taschen 26, 28, 30 mit Piktogrammen und Text bedruckt, die über den Inhalt der jeweiligen Tasche 26, 28, 30 Auskunft geben.

Die Rückenbahn 14, die Taschenbahnen 16, 18 und die Nähte 24 zusammen bilden jeweils Taschen 26, 28, 30 verschiedener Größe. Die Öffnungen 32 der Taschen 26, 28, 30 sind jeweils parallel zur Längsmittelachse der Rückenbahn 12 öffnend und einander gegenüberliegend angeordnet.

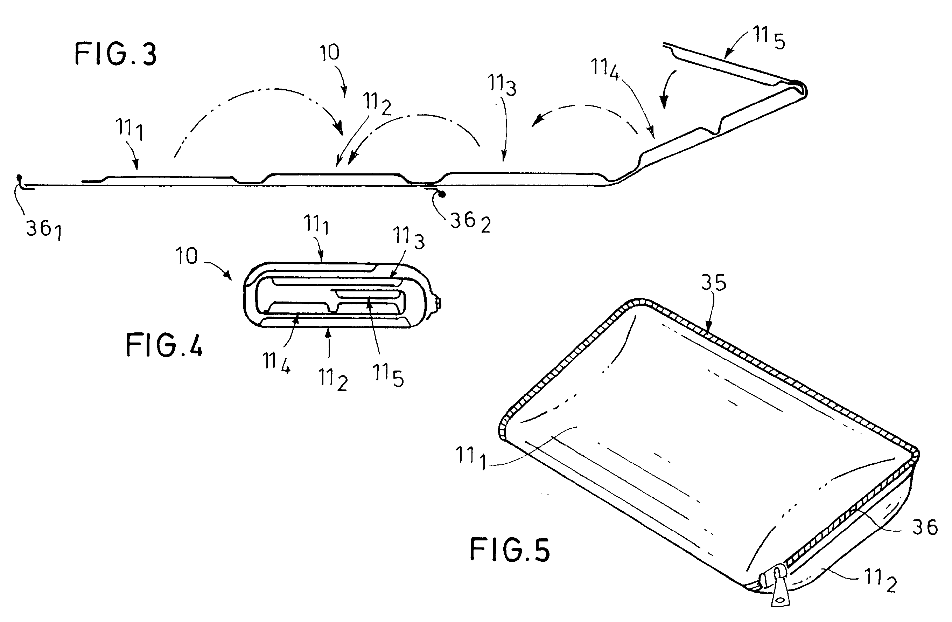

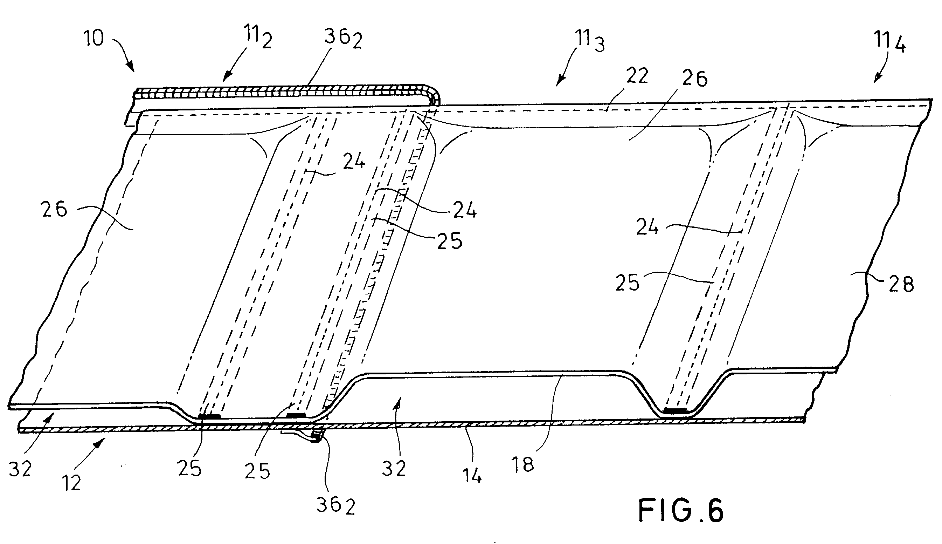

Die Nothilfepackung besteht aus insgesamt fünf Faltabschnitten 11₁ - 11₅, wobei die ersten beiden aneinandergrenzenden Faltabschnitte 11₁, 11₂ zu einer Schutzmappe 35 zusammengeklappt werden können, wie in den Fign. 4 und 5 dargestellt ist. Es können alternativ aber auch zwei andere aneinandergrenzende Faltabschnitte die Schutzmappe bilden.

Die übrigen drei Faltabschnitte 11₃, 11₄, 11₅ werden spiralartig zusammengefaltet, können jedoch auch zickzackartig zusammengefaltet werden, so dass sie von den beiden buchartig zusammengeklappten Faltabschnitten 11₁, 11₂ der Schutzmappe 35 sandwichartig umfasst werden.

Als Schließmittel ist auf der Rückseite der Rückenbahn 14 ein Reißverschluss 36 aufgenäht, dessen beide Hälften 36₁, 36₂ einen durchgehenden geschlossenen Kreis um die beiden Faltlagen 11₁, 11₂ herum bilden. Durch den Reißverschluss 36 werden alle drei offenen Seiten der Schutzmappe 35 dicht verschlossen. Die beiden Reißverschlusshälften 36₁, 36₂ werden durchgehend auf die Außenseite der Rückenbahn 14 aufgenäht, was einfach und preiswert vornehmbar ist.

Zwischen den jeweiligen Faltabschnitten 11₁ - 11₅ sind taschenlose Verbindungsabschnitte 13 vorgesehen, die einen Abstand zwischen den Faltabschnitten 11₁ - 11₅ herstellen. Dadurch wird ein Ineinanderfalten der Faltabschnitte 11₁ - 11₅ ermöglicht, auch wenn in den Taschen 26, 28, 30 Erste-Hilfe-Mittel von einer Höhe von 1 - 2 cm untergebracht sind.

Wie in Fig. 6 dargestellt, sind im Bereich der Nähte 24 schmale Verstärkungsstreifen 25 aus starkem Kunststoffmaterial auf die Taschenbahn 16, 18 aufgelegt und vernäht, wodurch das Einreißen der Taschenbahn 16, 18 im Bereich der Nähte 24 vermieden wird. Alternativ oder ergänzend kann an den randseitigen Nähten 20, 22 ein schmaler Streifen der Rückenbahn 14 auf die Taschenbahn 16, 18 umgeklappt sein, um als Verstärkungsstreifen zu dienen.

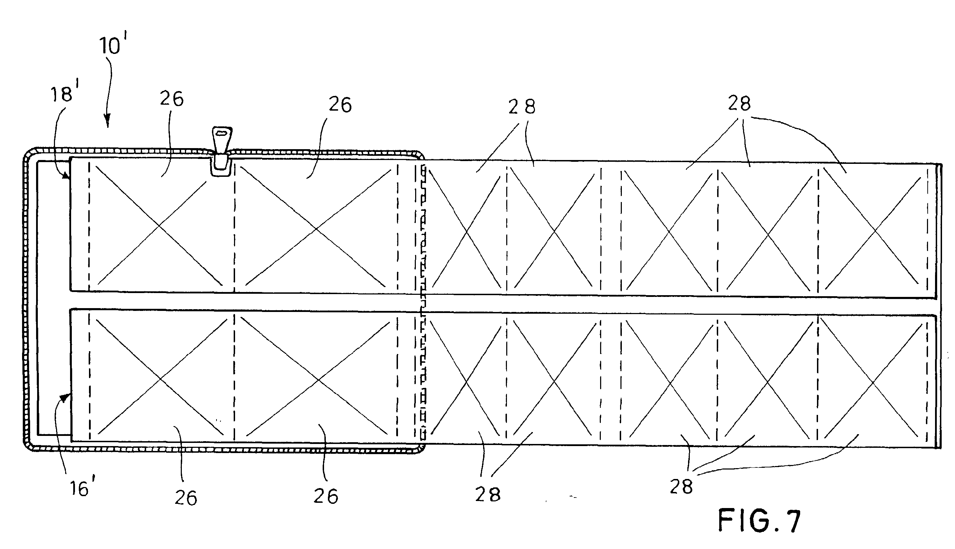

Die Nothilfepackung 10' der Fig. 7 entspricht der in den Fign. 1 - 6 dargestellten Nothilfepackung 10, mit dem Unterschied, dass die einander gegenüberliegenden Taschen 26, 28 jeweils von gleicher Länge und nicht versetzt zueinander angeordnet sind.

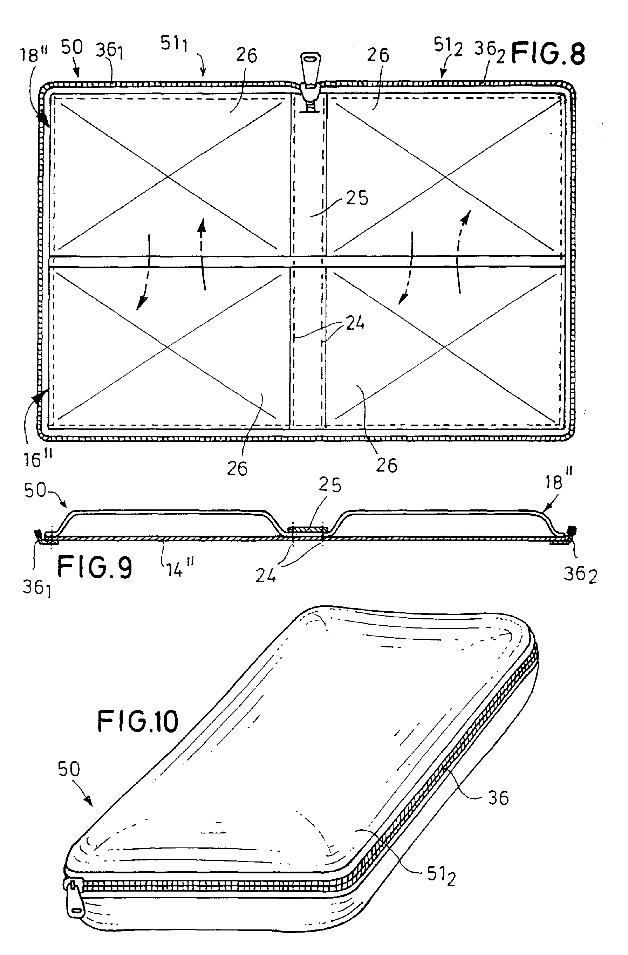

Die Nothilfepackung 50 der Fign. 8 - 10 unterscheidet sich von der Nothilfepackung der Fign. 1 - 6 dadurch, dass nur zwei Faltabschnitte 51₁, 51₂, jedoch keine weiteren Faltabschnitte vorgesehen sind. Die Taschenbahnen 16'', 18'' sind auf die Rückenbahn 14'' aufgenäht. Die beiden Faltabschnitte 51₁, 51₂ bilden zusammengeklappt ebenfalls eine Schutzmappe, in deren Innenraum die Taschen 26 geschützt angeordnet sind.

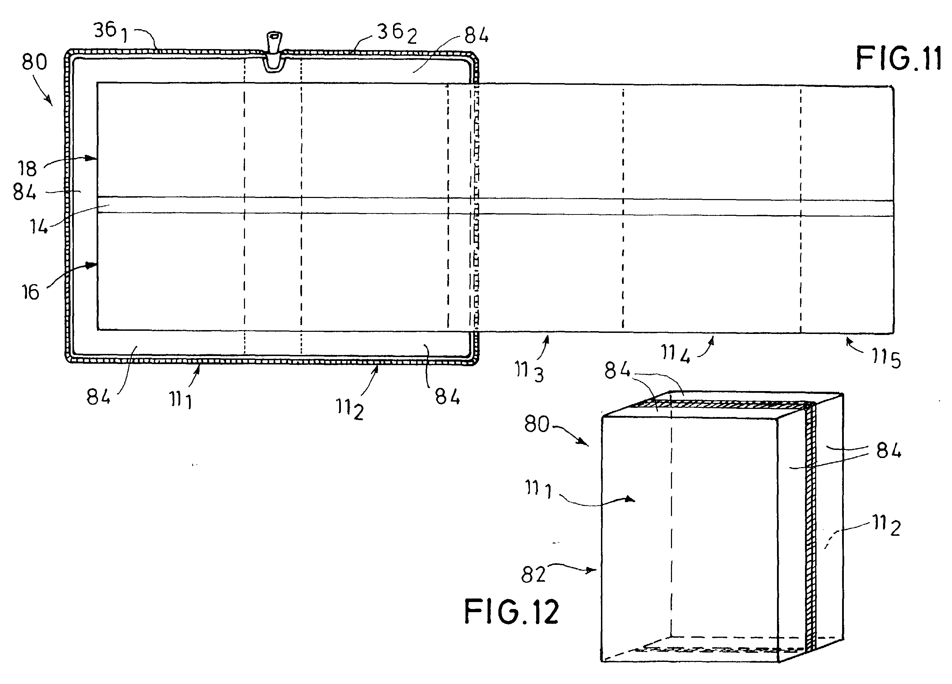

Die Nothilfepackung 80 der Fign. 11 und 12 weist gegenüber den Nothilfepackungen 10, 10' der Fign. 1 - 8 einen um die beiden die Faltmappe 82 bildenden Faltabschnitte 11₁, 11₂ umlaufenden Randstreifen 84 auf, durch den der Reißverschluss 36₁, 36₂ ca. 1 cm beabstandet zu der Rückenbahn 14 angeordnet ist. Der Randstreifen 84 bildet bei zusammengeklappter und geschlossener Faltmappe 82 drei Seitenwände der Faltmappe 82, wie in Fig. 12 dargestellt.

## Patentansprüche

1. Nothilfepackung zur Aufnahme von Erste-Hilfe-Mitteln, mit
einem flexiblen Rücken (12), der auf seiner Innenseite Taschen (26, 28, 30) für die Erste-Hilfe-Mittel aufweist und der in mindestens zwei Faltabschnitte (11₁ - 11₅) unterteilt ist,
wobei zwei aneinander angrenzende Faltabschnitte (11₁, 11₂) buchartig zusammenklappbar sind und zusammengeklappt eine Schutzmappe (35) bilden, in deren Innenraum die Taschen (26, 28, 30) geschützt angeordnet sind, und
wobei der Rücken (12) von einer durchgehenden Rückenbahn (14) gebildet ist,
**dadurch gekennzeichnet,**
**dass** die Taschen (26, 28, 30) auf der einen Seite von der Rückenbahn (14) und auf der anderen Seite von mindestens einer Taschenbahn (16, 18) begrenzt sind, und
**dass** das Taschenbahnmaterial von dem Rückenbahnmaterial verschieden ist.

2. Nothilfepackung nach Anspruch 1, **dadurch gekennzeichnet, dass** das Rückenbahnmaterial fester und dicker ist als das Taschenbahnmaterial.

3. Nothilfepackung nach Anspruch 1 oder 2, **dadurch gekennzeichnet, dass** die Rückenbahn (14) und die Taschenbahn (16, 18) mindestens drei Faltabschnitte (11₁ - 11₅) bilden, wobei zwischen die beiden Schutzmappen-Faltabschnitte (11₁, 11₂) die übrigen Faltabschnitte (11₃ - 11₅) einklappbar sind.

4. Nothilfepackung nach einem der Ansprüche 1 - 3, **dadurch gekennzeichnet, dass** der Rücken (12) von der durchgehend einlagigen Rückenbahn (14) gebildet wird und die Taschenbahn (16, 18) in Längsrichtung durchgehend einlagig ausgebildet ist, wobei die Taschenbahn (16, 18) auf der Innenseite der Rückenbahn (14) linienförmig befestigt ist.

5. Nothilfepackung nach einem der Ansprüche 1 - 4, **dadurch gekennzeichnet, dass** eine zweite Taschenbahn (18) parallel und gegenüberliegend der ersten Taschenbahn (16) vorgesehen ist, wobei die Taschenöffnungen (32) der Taschen (26, 28, 30) beider Taschenbahnen (16, 18) zur Längsmittelachse der Rückenbahn (14) öffnend und einander gegenüberliegend vorgesehen sind.

6. Nothilfepackung nach einem der Ansprüche 1 - 5, **dadurch gekennzeichnet, dass** die Rückenbahn (14) durchgehend einlagig ist und aus einem textilen Gewebe, insbesondere aus einem Kunstfasergewebe, besteht.

7. Nothilfepackung nach einem der Ansprüche 1 - 6, **dadurch gekennzeichnet, dass** die Taschenbahn (16, 18) aus einer einlagigen Kunststofffolie besteht.

8. Nothilfepackung nach einem der Ansprüche 1 - 7, **dadurch gekennzeichnet, dass** die Taschenbahn (16, 18) mit der Rückenbahn durch Vernähungen (22, 24) verbunden ist.

9. Nothilfepackung nach Anspruch 8, **dadurch gekennzeichnet, dass** im Bereich der Vernähungen (22, 24) Verstärkungsstreifen (25) zur Verstärkung der Vernähungen (22, 24) vorgesehen sind.

10. Nothilfepackung nach einem der Ansprüche 1 - 9, **dadurch gekennzeichnet, dass** ein Reißverschluss (36) vorgesehen ist, der die zwei zur Schutzmappe (35) zusammengefalteten Rückenbahn-Faltabschnitte (11₁, 11₂) an den drei offenen Seiten miteinander verbindet, so dass alle drei offenen Seiten der Schutzmappe (35) mit dem Reißverschluss (36) verschließbar sind.

11. Nothilfepackung nach einem der Ansprüche 5 - 10, **dadurch gekennzeichnet, dass** die Gewebe-Rückenbahn (14) auf ihrer Innenseite mit einer flüssigkeitsundurchlässigen Kunststoffbeschichtung versehen ist.
